# EUROPEAN PATENT SPECIFICATION

(11) **EP 1 644 069 B1**
(45) Date of publication and mention of the grant of the patent: **08.08.2007**
(21) Application number: 04777318.9
(22) Date of filing: 29.06.2004
(51) Int. Cl.: A61M 25/00, A61F 2/01, A61M 25/01, A61B 17/22

(54) **DEVICES FOR ASPIRATING FROM FILTERS**
VORRICHTUNGEN ZUR ASPIRATION AUS FILTERN
DISPOSITIFS POUR L'ASPIRATION A PARTIR DE FILTRES

(30) Priority: 02.07.2003 US 612719
(43) Date of publication of application: 12.04.2006
(73) Proprietor: Medtronic Vascular, Inc., Santa Rosa, CA 95403 (US)
(72) Inventor: PATEL, Mukund, San Jose, CA 95118 (US); NOOL, Jeffrey, San Jose 95716 (FJ)
(74) Representative: Bauer, Friedrich
(86) International application number: PCT/US2004/021042
(87) International publication number: WO 2005/004968

(56) References cited:
- US-A- 5 769 816
- US-A- 6 135 991
- US-A1- 2002 188 313
- US-A1- 2003 023 263

## Description

### Field of the Invention

The present invention generally relates to aspiration catheters for aspirating emboli, thrombi, and other particles from a blood vessel. The aspiration catheters described herein are particularly well adapted for removing particles from a filter device located in a blood vessel.

### Description of the Related Art

Medical catheters have proven efficacious in treating a wide variety of blood vessel disorders. Moreover, these types of catheters have permitted technicians to treat disorders with minimally invasive procedures that, in the past, would have required complex and perhaps life-threatening surgeries. In one example, a small inflatable balloon is provided along the distal end portion of a catheter body for use in a procedure commonly referred to as angioplasty. During this procedure, the balloon is advanced through a patient's vasculature to a stenotic lesion (i.e., a clogged artery) and the balloon is inflated to reopen the vessel.

Angioplasty is often performed to treat a medical condition known as coronary heart disease. This disease is characterized by plaque buildup (i.e., atherosclerosis) in one or more coronary arteries. The plaque buildup impedes the flow of blood and thereby deprives the heart tissue of sufficient oxygenated blood. It is critical to treat a narrowed coronary artery as soon as the problem is diagnosed because the constriction may become further occluded by the formation of thrombi (i.e., blood clots) along the roughened surfaces of the plaque. Worse yet, a coronary artery may become completely occluded when a blood clot or other emboli lodges in the constriction. When this happens, myocardial infarction can occur, often resulting in sudden cardiac death.

In addition to angioplasty, other intervention procedures are commonly performed for treating occluded blood vessels. Other procedures include atherectomy, stent deployment, introduction of specific medication by infusion, and bypass surgery.

Although catheter-based intervention procedures have met with considerable success for treating coronary heart disease and other blood vessel disorders, there are still a variety of dangers associated with these procedures. In particular, there is a substantial risk that embolic particles may become dislodged or liberated from the inner wall of the vessel during treatment The liberated embolic particles can migrate through the circulatory system and block another blood vessel, possibly leading to ischaemic events, such as infarction or stroke.

Due to the considerable dangers associated with the dislodgment of embolic particles during treatment, additional catheter-based medical devices have been developed for capturing and removing the dislodged embolic particles from the bloodstream. For example, one type of catheter-based medical device adapted for this purpose comprises a vascular filter that is mounted on the distal end portion of a guidewire. Filters of this type typically comprise a blood-permeable element that may take a wide variety of forms, such as, for example, a porous material, a plurality of struts or a perforated basket

During use, the guidewire is used to advance the vascular filter to a location distal to the treatment site before treating the blood vessel. After being advanced, the filter is expanded to engage the inner wall of the vessel such that most or all of the blood in the treated vessel flows through the filter structure. As a result, liberated embolic particles are captured and contained within the vascular filter. After the filter is deployed in the blood vessel, the guidewire is typically used for directing a therapy catheter (e.g., angioplasty balloon catheter) to the treatment site. After the treatment is concluded and the therapy catheter is removed, the vascular filter is collapsed and withdrawn to contain and remove the captured embolic particles.

US 2003/0023263 A1 discloses an aspiration catheter according to the preamble of claim 1 wherein the distal port of the guidewire lumen opens into the interior of a distal portion of the catheter body.

Although vascular filters are generally effective tools for capturing and removing emboli, in practice, it has been found that a variety of shortcomings significantly limit their effectiveness. For example, a vascular filter may become filled with embolic material during the interventional procedure to the point where the blood flow through the filter is significantly reduced or stopped completely. This is a serious problem because, in order to capture particles, there needs to be a continuous flow (i.e., perfusion) of blood through the filter. If the flow of blood stops due to clogging of the filter, embolic particles will remain suspended in the blood proximal (i.e. upstream) of the filter. When the clogged filter is collapsed and removed, the flow of blood will then resume through the vessel and the suspended embolic particles will migrate downstream where they may produce serious complications.

In another shortcoming, a vascular filter that is full of embolic particles may be very difficult to remove from a patient's vasculature in a safe and effective manner. In particular, as the filter is collapsed, the embolic particles trapped within the filter may become dislodged or otherwise pushed out of the filter, thereby releasing the embolic particles back into the blood vessel.

In yet another related shortcoming, a vascular filter that is full of embolic particles may be very difficult to retract through a vascular stent that has been deployed in a blood vessel. The large profile of a full filter and the delicate structure of the filter material can make it very difficult, or sometimes impossible, to retract the filter through the deployed stent. Serious complications can arise if the filter material becomes entangled with the stent structure.

In summary, vascular filters are useful tools for capturing and removing embolic particles from the bloodstream during interventional procedures. However, a filter that has become full of embolic particles may present serious difficulties that can limit the effectiveness and safety of the associated medical procedure. Accordingly, there is an urgent need for a new device for removing embolic particles from a blood vessel. It is desirable that embodiments of such a device be adapted for removing embolic particles from a vascular filter while the filter is deployed within a blood vessel. It is also desirable that such a device be reliable, convenient to use and relatively inexpensive to manufacture. Such a device would significantly improve the efficacy and safety of blood vessel treatments by ensuring adequate perfusion of blood through the filter and by facilitating the removal of the filter from the patient's vasculature.

### Summary of the Invention

Various embodiments of the present invention provide aspiration catheters for aspirating emboli or other particles from a blood vessel. Certain embodiments are particularly well adapted for removing particles from a vascular filter in a blood vessel. Accordingly, the devices are well adapted for use with medical treatments, such as angioplasty, wherein embolic particles may dislodge and migrate through the bloodstream.

According to claim 1, the aspiration catheter comprises an elongate catheter body formed with an aspiration lumen and a guidewire lumen, wherein the aspiration lumen extends distally beyond the distal end of the guidewire lumen and the distal port of the guidewire lumen opens to the exterior of the elongate catheter body.

In another embodiment, an aspiration catheter system is provided. The system includes the invention aspiration catheter, a guidewire and a vascular filter disposed along the distal end portion of the guidewire. It should be noted that, in this embodiment, the distal segment allows the aspiration port to be advanced beyond the proximal end (i.e., proximal hub) of the filter.

The inventive aspiration catheter can be used in a method for treating a blood vessel, wherein the guidewire is delivered transluminally through the blood vessel until the distal end is located in a desired location, typically distal to a treatment site. The expandable member is then expanded within the blood vessel such that the expandable member at least partially forms an interior volume adapted to retain particles therein. The aspiration catheter is advanced over the guidewire until the aspiration port is located within an interior volume of the expanded expandable member. A negative pressure is applied to the proximal connector of the aspiration catheter. The negative pressure is preferably applied while the aspiration port is positioned within the interior volume of the expandable member for drawing particles from the interior volume and out of the blood vessel.

### Brief Description of the Drawings

**FIGURES 1A-1G** illustrate an exemplifying medical treatment procedure wherein embolic particles are dislodged and captured by an expandable filter during an interventional procedure.

**FIGURE 2** is a side view of one embodiment of an aspiration catheter including a distal segment wherein the aspiration lumen extends beyond the distal guidewire port.

**FIGURE 3** is a cross-sectional view of the aspiration catheter of FIGURE 2 shown through line 3-3.

**FIGURES 4A-4D** are enlarged side views illustrating various distal segment embodiments for use with an aspiration catheter.

**FIGURES 5A-5C** are side views illustrating a method of aspirating embolic particles using the aspiration catheter of **FIGURE 2**.

**FIGURE 6** is a side view illustrating a method of aspirating embolic particles from the interior volume of an alternative type of vascular filter formed with proximal inlet holes.

**FIGURE 7** is a side view illustrating a method of aspirating embolic particles from the interior volume of a basket-shaped vascular filter.

**FIGURE 8** is a side view illustrating a method of aspirating embolic particles from the interior volume of a vascular filter shaped for use with an aspiration catheter.

**FIGURE 9** is a side view of an aspiration catheter not being part of the invention and provided with a side port for slidably receiving a guidewire.

**FIGURE 10** is a side view illustrating the aspiration catheter of **FIGURE 9** in combination with a filter guidewire.

**FIGURE 10A** is a cross-sectional view of the aspiration catheter and guidewire of **FIGURE 10.**

**FIGURE 11** is a side view of another alternative embodiment wherein the aspiration catheter further includes an irrigation lumen for flushing particles from the interior volume of a vascular filter.

**FIGURE 12** is a cross-sectional view of the embodiment of **FIGURE 11** shown through line 12-12.

**FIGURE 13** is a side view illustrating the aspiration catheter of **FIGURE 11** in combination with a filter guidewire.

**FIGURE 14** is a side view of another alternative embodiment of an aspiration catheter modified to include a plurality of side aspiration ports along a distal segment

**FIGURE 15** is a side view of another alternative embodiment of an aspiration catheter wherein the guidewire lumen extends to the proximal end of the catheter.

**FIGURE 16** is a side view of another alternative embodiment of an aspiration catheter further comprising an inflatable balloon disposed along the elongate catheter body.

**FIGURE 17** is a cross-sectional view of the embodiment of **FIGURE 16** shown through line 17-17.

**FIGURE 18** is a side view illustrating a method of aspirating emboli from a filter while performing treatment on a lesion using the aspiration catheter of **FIGURE 16.**

### Detailed Description of the Preferred Embodiments

Various embodiments of the present invention depict aspiration catheters and methods of use that are well-suited for removing embolic particles from vascular filters. It should be appreciated that the principles and aspects of the embodiments disclosed and discussed herein are also applicable to other devices having different structures and functionalities. For example, certain structures and methods disclosed herein may also be applicable to various other types of aspiration, irrigation and delivery catheters. Furthermore, certain embodiments may also be used in conjunction with other medical devices or other procedures not explicitly disclosed. However, the manner of adapting the embodiments described herein to various other devices and functionalities will become apparent to those of skill in the art in view of the description that follows.

### I. OVERVIEW OF VASCULAR FILTERS AND METHODS OF USE

Vascular filters are commonly used for providing distal protection against embolization in conjunction with a diagnostic or therapeutic procedure. Vascular filters are typically provided along the distal end portion of a guidewire or other elongate body and are adapted for placement within a blood vessel. Vascular filters are deployed (i.e., expanded) at a location distal (i.e., downstream) of a treatment site prior to performing the diagnostic or therapeutic procedure. The filters are configured for capturing embolic particles that may become dislodged from the vessel wall during treatment of the blood vessel.

The term "embolic particles" as used herein primarily refers to pieces of atherosclerotic plaque that may become dislodged from the vessel wall during treatment. However, the term "embolic particles" may also include a variety of other substances, such as, for example, blood clots, fat globules, or small pieces of body tissue. The term "occlusion" as used herein generally refers to a narrowing in a blood vessel, sometimes referred to as a stenosis. An occlusion may result from atherosclerosis, fibromuscular dysplasia, scar formation or other factors.

The term "guidewire" as used herein is intended to broadly include guidewires and other similar and elongate members. The terms "vascular filter," or simply "filter," as used herein are broad terms that include a wide variety of devices adapted to prevent the migration of embolic particles through a blood vessel. The term "filter guidewire" as used herein generally refers to a medical device comprising a filter mounted on the distal end of a guidewire. The term "distal protection" as used herein refers to protecting the portion of the blood vessel downstream of the treatment site. It will be appreciated that other distal protection devices may be used instead of a filter without departing from the scope of the invention. The term "interior volume" as used herein generally refers to the partially enclosed interior region of the filter or expandable member adapted for containing embolic particles. The interior volume is typically defined by the interior region between the proximal and distal ends of the filter, but should be considered to include any portion of the filter capable of capturing and containing embolic particles.

In recent years, a variety of different vascular filters have been proposed for capturing embolic particles from a bloodstream. For example, certain types of filters are provided in the form of a braided wire or a non-permeable material formed with perforations. In other examples, vascular filters may take the form of struts, sometimes covered with a porous material or membrane. Vascular filters adapted for capturing embolic particles typically have a pore-size of about 100 microns. However, the pore-size may vary from about 50 to 250 microns, depending on the location and purpose of the filter. In addition, a single filter may be provided with a plurality of pores having a variety of different sizes.

In cases where the treatment site is located in tortuous vessels that are remote from the vascular access point, such as coronary arteries, a steerable guidewire may be used in conjunction with a filter. In various embodiments, a filter may be self-expanding or mechanically deployable. In a mechanically deployable variation, a filter may include a pull wire and have proximal and distal ends that are movable relative to one another to expand or collapse the filter. To facilitate visualization under fluoroscopy, a filter may be used in combination with a radiopaque marker. Examples of filters that are currently commercially available on the market include the Medtronic Interceptor^{™}, the Boston Scientific EPI^{™}, and the Cordis Angioguard^{™}.

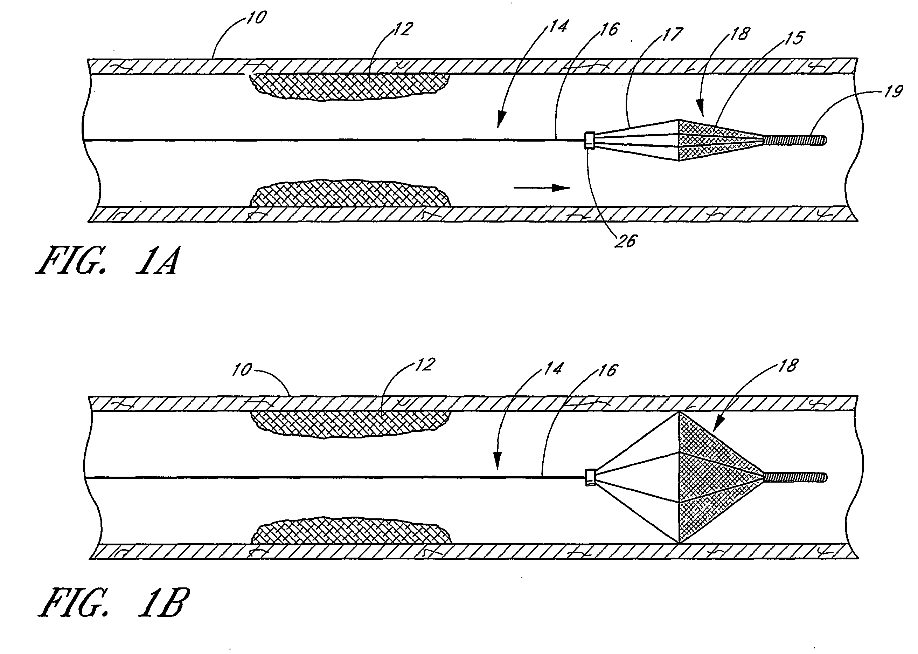

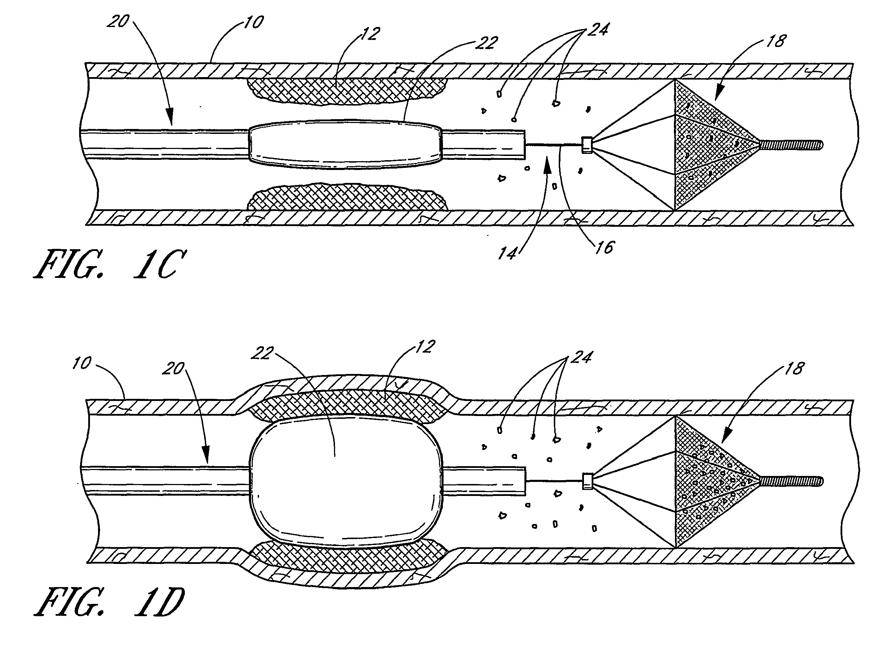

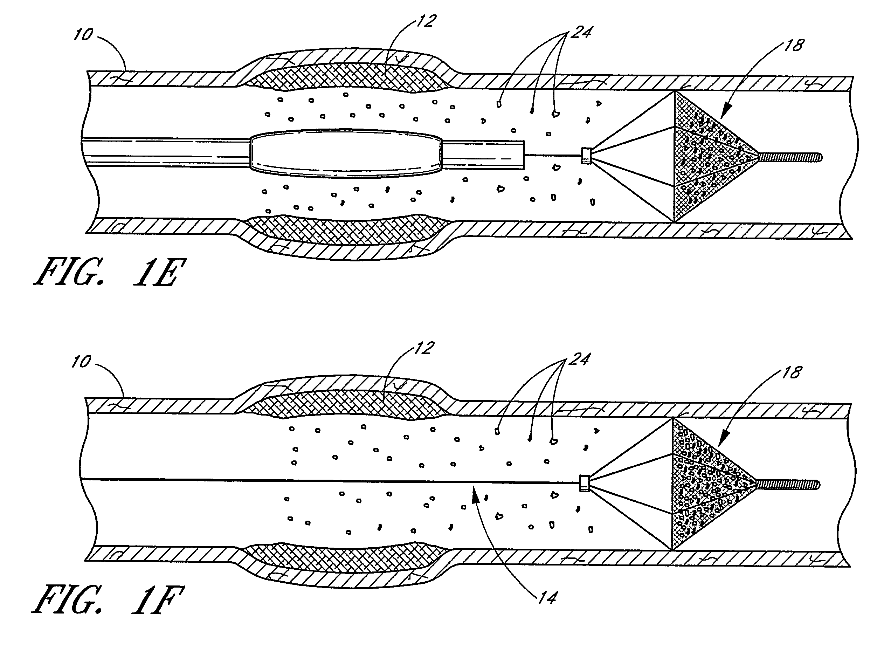

For purposes of illustration, **FIGURES 1A-1G** shown a series of steps involving a transluminal medical procedure wherein an occlusion in a blood vessel is treated using a therapy catheter to increase the flow of blood through the vessel. The illustrated medical procedure, commonly referred to as angioplasty, generally involves expanding an inflatable balloon at the treatment site to dilate the occlusion. Prior to the procedure, a vascular filter is deployed downstream of the occlusion for use as a distal protection device. During the procedure, as embolic particles become dislodged from the inner wall of the blood vessel, the particles are captured within an interior volume of the vascular filter. Although one particular filter embodiment is illustrated for use in conjunction with this procedure, a wide variety of other filters embodiments may be used.

Similarly, although one particular type of therapy catheter is illustrated in the example, a wide variety of other therapy catheters may be used to treat a blood vessel in similar medical procedures. For example, a thermal balloon may be used at a treatment site for applying heat to "mold" the vessel to the size and shape of the angioplasty balloon. In another procedure, a therapy catheter may be used to deliver an intravascular stent within a blood vessel to keep the vessel open. Still further, cutting, shaving, scraping or pulverizing devices can be delivered to excise the occlusion in a procedure known as atherectomy. A laser or ultrasound device can also be delivered and used to ablate plaque in the vessel. Thrombectomy devices can be used, as can rheolitic devices and devices that create a venturi effect within the artery. Various thrombolytic or other types of drugs can be delivered locally in high concentrations to the site of the occlusion. It is also possible to deliver various chemical substances or enzymes via a drug delivery catheter to the site of the stenosis to dissolve the obstruction. Accordingly, the term "therapy catheter" as used herein encompasses these and a wide variety of other devices.

Referring now in detail to **FIGURE 1A**, diseased blood vessel 10 is shown having occlusion 12 that restricts the flow of blood through the vessel. Occlusion 12 is produced by atherosclerotic plaque that has built up along the inner wall of vessel 10. Before treating the occlusion, filter guidewire 14 is advanced through blood vessel 10, as illustrated in **FIGURE 1A**. Filter guidewire 14 comprises guidewire 16 with expandable vascular filter 18 mounted on the distal end. Expandable vascular filter 18 generally includes frame 17 comprising a plurality of struts and blood permeable element 15, such as a mesh, attached to the frame. Frame 17 attaches to guidewire 16 at proximal filter hub 26. Proximal filter hub 26 is located at the distal end of guidewire 16. The illustrated embodiment of filter guidewire 14 further includes atraumatic tip 19 at the extreme distal end for minimizing damage to the vessel intima during advancement. Vascular filter 18 is placed in blood vessel 10 to provide distal protection during the therapeutic procedure. In the illustration, the flow of blood through vessel 10 is moving from left to right as indicated by an arrow.

Referring now to **FIGURE 1B**, filter 18 is expanded within blood vessel 10 such that the outer periphery of filter 18 engages the inner wall of blood vessel 10. Filter 18 may be self-expanding as delivered through an outer sheath or mechanically deployed (e.g., actuated with a pull wire). Expanded filter 18 has an interior volume adapted to capture embolic particles from the blood as the blood passes through the filter. More details regarding filter guidewires used for distal embolic protection may be found in Assignee's U.S. Patent No. 6,312,407, U.S. Application No. 09/918,441, filed July 27, 2001, and U.S. Application No. 10/099,399, filed March 15, 2002.

Referring now to **FIGURE 1C**, therapy catheter 20 is advanced over guidewire 16 to the site of occlusion 12. In the illustrated example, therapy catheter 20 is an elongate tubular body having inflatable angioplasty balloon 22 mounted along the distal end portion. As illustrated, balloon 22 is positioned at the site of occlusion 12. During the advancement of balloon 22 into the occlusion, embolic particles 24, typically in the form of small pieces of plaque, may become dislodged or otherwise liberated from the wall of blood vessel 10. Embolic particles 24 migrate downstream with the blood and are captured and contained within the interior volume of filter 18.

Referring now to **FIGURE 1D,** with inflatable balloon 22 positioned at occlusion 12, balloon 22 is inflated to stretch the diseased portion of the vessel wall and partially compress the plaque, thereby dilating the passageway through blood vessel 10. According to various known techniques, balloon 22 can be inflated with any suitable inflation medium such as, for example, a dilute x-ray contrast liquid. During dilation of the stenosis, additional particles 24 are dislodged and flow through blood vessel 10 until they are captured in the interior volume of filter 18.

Referring now to **FIGURE 1E**, after the therapeutic procedure is completed, balloon 22 is deflated. As illustrated, additional particles 24 that were previously trapped between balloon 22 and the inner wall of vessel 10 are released into the bloodstream. Additional particles 24 are captured within the interior volume of filter 18. Referring now to **FIGURE 1F**, therapy catheter 20 is removed from blood vessel 10. Finally, as illustrated in **FIGURES 1G**, filter 18 is collapsed to prepare filter guidewire 14 and the captured particles for removal from blood vessel 10.

As illustrated in **FIGURES 1A-1G,** a large quantity of embolic particles may become dislodged from a vessel wall during the treatment of a blood vessel. When the quantity and size of liberated embolic particles is substantial, a vascular filter may become partially or completely filled with material, causing serious problems. In a first problem, a clogged filter obstructs blood flow, which may cause ischaemia, associated pain and possible infarction in the downstream tissues. In a second problem, when the filter becomes filled, the perfusion of blood through the filter is significantly reduced or stopped and the filter becomes unable to trap additional particles from the blood. As a result, dislodged particles may remain suspended in the blood at a location proximal (i.e., upstream) of the filter. As illustrated in **FIGURE 1G**, when the filter is collapsed and removed, particles 24 remaining in the blood proximal to the filter will become free to migrate downstream through the blood where they may cause ischaemic events. In a third related problem, as an over-filled filter is collapsed for removal, embolic particles in the filter may be ejected through the inlet opening(s) or "squeezed through" the filter pores and released back into the blood stream, at least partially defeating the intended purpose of the filter.

Aspiration catheters have been proposed to help remove embolic particles from the bloodstream before removing the filter from the blood vessel. After the therapeutic procedure is concluded, a therapy catheter may be exchanged with an aspiration catheter for this purpose. Further details of this exchange, as well as other details regarding treatment procedures, are described in Assignee's U.S. Patent 6,544,276 and U.S. Patent 6,135,991.

Although various types of aspiration catheters are configured for aspirating particles from the region of the blood vessel proximal to the vascular filter, these aspiration catheters may not always be well adapted for removing particles from within the interior volume of a vascular filter. Accordingly, a need exists for a new and improved aspiration catheter adapted for removing particles from within the interior volume of a vascular filter or other substantially occlusive device deployed in a blood vessel.

### II. IMPROVED ASPIRATION CATHETERS AND METHODS OF USE

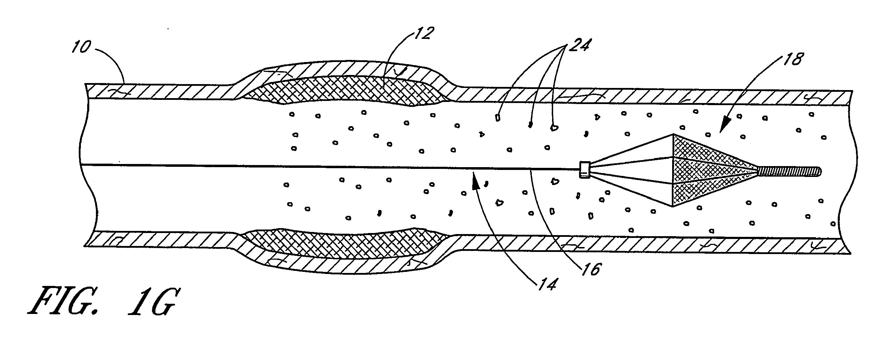

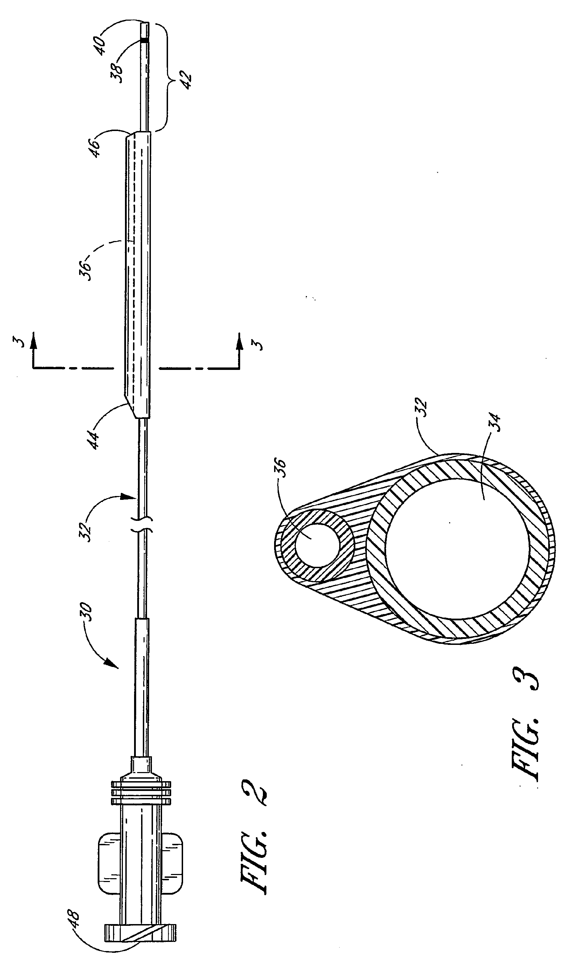

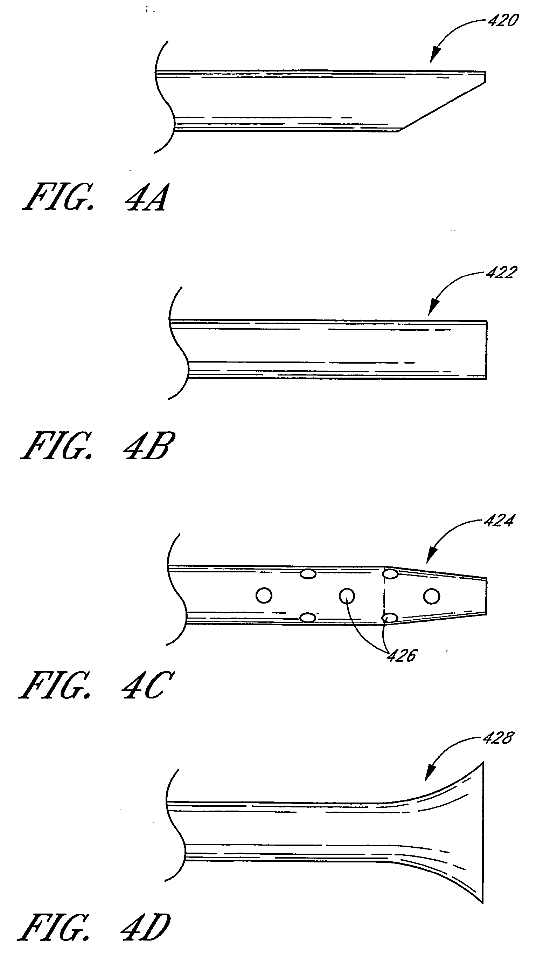

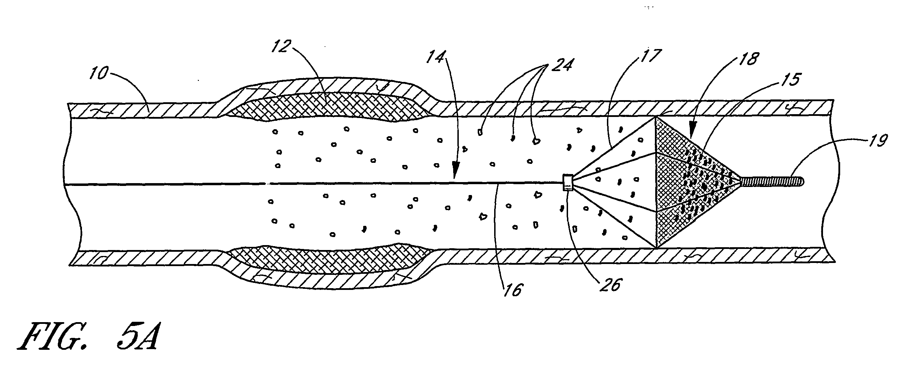

Referring now to **FIGURES 2** and **3**, for purposes of illustration, one embodiment of improved aspiration catheter 30 adapted for removing particles from a blood vessel includes, generally, elongate catheter body 32 formed with aspiration lumen 34 and guidewire lumen 36. Aspiration lumen 34 may extend the entire length of elongate catheter body 32 from proximal connector 48 to aspiration port 40. Guidewire lumen 36 may extend only a short length from proximal guidewire port 44 to distal guidewire port 46 along the distal end portion of catheter body 32.

Aspiration catheter 30 is sized for advancement through a patient's vasculature. In various embodiments, aspiration lumen 34 may have a length of about 120 to 300 cm. The illustrated aspiration catheter includes radiopaque marker 38 along the distal end portion to aid in locating the device during advancement to a treatment site. In one embodiment, the distal end portion of aspiration catheter 30 is constructed of a soft material to prevent damage to the inner wall of the blood vessel during advancement there through.

As illustrated, aspiration lumen 34 and guidewire lumen 36 are located adjacent one another along a distal end portion of aspiration catheter 30. The aspiration lumen and guidewire lumen may be provided as separate structures or may be integrally formed into a single catheter body. In one embodiment, aspiration lumen 34 is substantially unobstructed for maximum efficiency and has a generally circular cross-section extending from the proximal end to the distal end. Alternatively, aspiration lumen 34 may have a crescent-shaped or oval-shaped cross-section. These and other shapes for the aspiration lumen are disclosed in Assignee's U.S. Application Serial No. 10/125,180, filed April 16, 2002. Various embodiments of aspiration lumen 34 may have an inner diameter between about 0.762 to 1.778 mm (0.03 to 0.07 inches). Proximal connector 48 is provided at the proximal end of elongate catheter body 32. Proximal connector 48 may be used for connecting a negative source of pressure to aspiration lumen 34.

In one embodiment, elongate catheter body 32 includes distal segment 42 wherein aspiration lumen 34 extends distally beyond distal guidewire port 46. Accordingly, distal segment 42 advantageously provides the ability to advance aspiration port 40 substantially beyond a guidewire element, such as filter hub 26, which may obstruct further advancement of guidewire port 46. As a result, aspiration catheter 30 is particularly well-suited for use in conjunction with a filter guidewire. By selecting distal segment 42 having a desired length, aspiration port 40 may be advanced beyond the proximal hub of the filter and into the interior volume of the filter for effective removal of particles therefrom. In one embodiment, distal segment 42 is about 2 mm to 30 mm in length. Distal segment 42 is preferably at least as long as the distance from the proximal filter hub to the distal end of the filter. Different filter designs will, of course, vary in length. A relatively short filter may be efficiently aspirated with an embodiment of aspiration catheter 30 having distal segment 42 of about 1.0 cm or shorter in length. Alternative "deep-basket" or "sock-shaped" filters may require an embodiment of aspiration catheter 30 having distal segment 42 of about 4.0 cm or longer. Thus, it will be appreciated that the length of distal segment 42 can be varied according to any particular need while remaining within the scope of the invention.

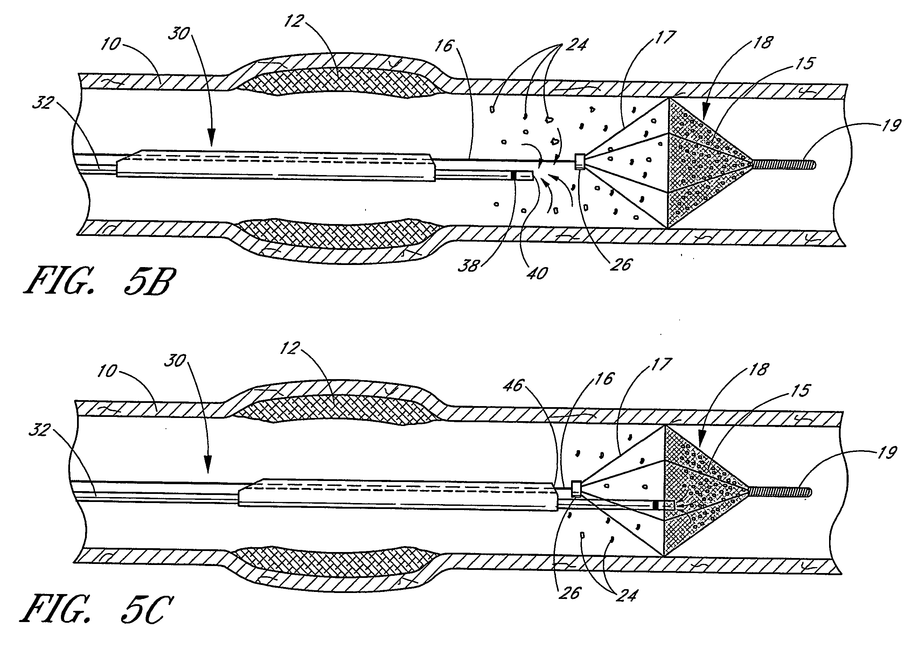

In various embodiments, aspiration port 40 at the end of distal segment 42 is angled or oblique to maximize the aspiration area and provide effective removal of particles. The oblique shape also reduces the possibility of the aspiration port sucking onto a vessel wall wherein it can cause trauma to or disruption of the vessel intima. Alternatively, or in addition to aspiration port 40, one or more side ports (see ports 426 in **FIGURE 4C**, as described below) may be provided along distal segment 42 of elongate catheter body 32 to enhance or increase the flow of blood and embolic particles 24 into aspiration lumen 34.

As discussed above, guidewire lumen 36 in aspiration catheter 30 is adapted to slidably receive a guidewire. If desired, a slit (not shown) may be provided along the side wall adjacent guidewire lumen 36 to facilitate insertion and removal of the guidewire. As illustrated, guidewire lumen 36 is relatively short as compared with the length of aspiration catheter 30. In various embodiments, guidewire lumen 36 may have a length of about 30 cm or less, and more preferably about 6 cm or less. Therefore, only a small portion of illustrated "single operator" aspiration catheter 30 rides over the guidewire during delivery to a treatment site.

**FIGURE 3** illustrates the cross-section of the aspiration catheter illustrated in **FIGURE 2** as shown through line 3-3. The cross-section illustrates the relative locations of aspiration lumen 34 and guidewire lumen 36 along elongate catheter body 32.

As will be appreciated by those skilled in the art, elongate catheter body 32 of aspiration catheter 30 should have sufficient structural integrity, or "stiffness," to permit the aspiration catheter to be pushed through the vasculature to distal arterial locations without buckling or undesirable bending. It is also desirable, however, for aspiration catheter 30 to be fairly flexible near its distal end, so that the aspiration catheter may be navigated through tortuous blood vessel networks.

In one preferred embodiment, aspiration catheter 30 is formed from a polymer such as polyethylene or PEBAX ® poly-ether-block co-polyamide and may have a variable stiffness along its length, with the proximal portion of the aspiration catheter being less flexible than the distal portion of the aspiration catheter. An aspiration catheter of this construction advantageously enables a physician to more easily insert the aspiration catheter into vascular networks that are otherwise difficult to access using conventional catheters of uniform stiffness. This is because the stiffer proximal portion provides the requisite structural integrity needed to advance the aspiration catheter without buckling, while the more flexible distal region is more easily advanced into and through tortuous blood vessel passageways.

In one preferred embodiment, variable stiffness along the length of aspiration catheter 30 is achieved by forming a polymeric aspiration catheter that incorporates a reinforcement along its length. For example, the aspiration catheter may be provided with a reinforcing braid or coil incorporated into its wall structure. The reinforcement can be formed of metal or of various polymers. To achieve variable stiffness, the distal region of the catheter may be provided with a braid or coil having a higher braid or coil density than that present in the braid or coil of the proximal region. The lower braid density in the proximal region makes it less flexible, or "stiffer," than the distal region of the catheter. Additional details regarding materials and methods of construction can be found in Assignee's U.S. Application Serial No. 10/125,180, filed April 16, 2002.

**FIGURES 4A-4D** illustrate various embodiments of a distal segment adapted for use with aspiration catheters described herein. Each of the illustrated distal segment embodiments includes an aspiration port at the tip. Accordingly, the shape of the tip defines the shape of the aspiration port. **FIGURE 4A** shows a distal segment having angled or oblique tip 420 for maximizing the aspiration area and to provide for effective retrieval of particles 24. Oblique distal tip 420 also minimizes the risk of the aspiration catheter sucking on the vessel wall that can cause trauma to or disruption of the vessel intima. The angle can be from about 5 degrees to about 90 degrees; however, an angle of about 25 degrees is preferred. **FIGURE 4B** shows a distal segment having blunt tip 422. In this embodiment, the aspiration port is not angled, but may be formed with a rounded surface to reduce vessel trauma. **FIGURE 4C** shows a distal segment having tapered tip 424 that also includes a plurality of side ports 426. Side ports 426 may be included with any of these or other distal segment embodiments. In one variation, one or more side ports may be used as an alternative to an aspiration port at the distal end. FIGURE 4D shows a distal segment having flared tip 428 adapted for providing an aspiration flow over a wider area. Flared tip 428 may be desirable for facilitating the aspiration of larger particles.

**FIGURES 5A-5C** will now illustrate an operational sequence using the embodiment of aspiration catheter 30 described above with reference to **FIGURES 2** and 3. In the illustrated operation, aspiration catheter 30 is used to remove particles 24 from vascular filter 18 after a therapeutic procedure has been performed on blood vessel 10. For clarity purposes, filter guidewire 14 and blood vessel 10 are indicated using the same reference numbers that were previously used with reference to **FIGURES 1A-1G.**

Referring now in detail to **FIGURE 5A**, filter guidewire 14 comprises vascular filter 18 mounted at the distal end of guidewire 16 for providing distal protection in blood vessel 10 during a therapeutic procedure. It should be noted that, in the illustrated sequence, there is no inflatable balloon or other occlusive device located proximal to the treatment site. Therefore, blood flows distally through vessel 10 toward filter 18. As described above, vascular filter 18 generally comprises frame 17 and blood permeable element 15. Frame 17 attaches to guidewire 16 at proximal filter hub 26, which defines a termination point at which a closely fitted guidewire lumen may no longer be advanced over the guidewire.

As illustrated, the interior volume of vascular filter 18 has become filled with embolic particles 24 due to the liberation of plaque from occlusion 12 during the therapeutic procedure. As a result, filter 18 has become clogged with particles to the point wherein the flow of blood through the filter (and hence the vessel) has diminished significantly. Because the flow of blood through the filter has diminished, a number of embolic particles 24 may remain in the region within vessel 10 proximal to filter 18. However, it will be appreciated that filter 18 need not be fully clogged to perform the sequence described below.

Referring now to **FIGURE 5B,** aspiration catheter 30 is advanced through the patient's vasculature over guidewire 16. In one mode of operation, aspiration catheter 30 may first be advanced to a position wherein aspiration port 40 is located just proximal of proximal filter hub 26. A radiopaque marker (not shown) is provided along the distal end of guidewire 16 at a location just proximal of filter 18. Additional radiopaque marker 38 is provided along the distal end portion of aspiration catheter 30 for facilitating alignment of aspiration catheter 30 and guidewire 16. Aspiration catheter 30 is advanced until radiopaque marker 38 on aspiration catheter 30 is located just proximal to the radiopaque marker on the guidewire. Preferably, the distance between the markers should not be greater than about 0.5 cm.

After aspiration catheter 30 has been advanced to the desired location, a source of negative pressure may be connected to the proximal connector (shown as element 48 in Figure 2) for creating an aspiration flow through the aspiration lumen. In one embodiment, aspiration may be started by opening a valve between connector 48 and a syringe or other vacuum source. The negative pressure may be applied while moving aspiration catheter 30, or while maintaining aspiration catheter 30 in a stationary position.

Embolic particles from the region of the blood vessel proximal to filter 18 are drawn into aspiration port 40. In addition, particles trapped within the filter itself may become dislodged and removed from the filter through aspiration port 40. The aspiration flow may be continued until adequate removal of embolic particles and/or adequate perfusion of blood through the filter has been reestablished. The valve may then be closed to stop the aspiration flow.

Referring now to **FIGURE 5C**, in an additional or alternative mode of operation, aspiration catheter 30 is advanced further over guidewire 16 until aspiration port 40 is located within the interior volume of vascular filter 18. This is possible in the illustrated embodiment because aspiration port 40 is located distal to distal guidewire port 46. Accordingly, distal guidewire port 46 may be located proximal to filter hub 26 while aspiration port 40 is located within the interior volume of filter 18. The advancement of aspiration port 40 into the interior volume can occur before, during, or after negative pressure is applied to the aspiration lumen. In any case, embolic particles are aspirated from the interior volume of filter 18 and out of blood vessel 10 through the aspiration lumen. To further enhance the removal of embolic particles from the filter, it may be desirable to move aspiration port 40 back and forth within the interior volume of filter 18. Furthermore, it may be desirable to advance aspiration catheter 30 forward to the point wherein aspiration port 40 comes into contact with filter 18 and/or the embolic particles contained therein. When aspiration port 40 is in contact with filter 18, the angled shape of aspiration port 40 advantageously reduces the possibility of the aspiration port grabbing onto the filter structure.

The sequence illustrated in **FIGURES 5A-5C** is particularly useful for removing particles 24 from a partially or fully clogged filter to increase the perfusion of blood through the filter and to remove particles 24 before collapsing and removing the filter. When used with a partially clogged filter, it may be advantageous to advance aspiration port 40 into the interior volume of filter 18 before connecting and activating the negative pressure source.

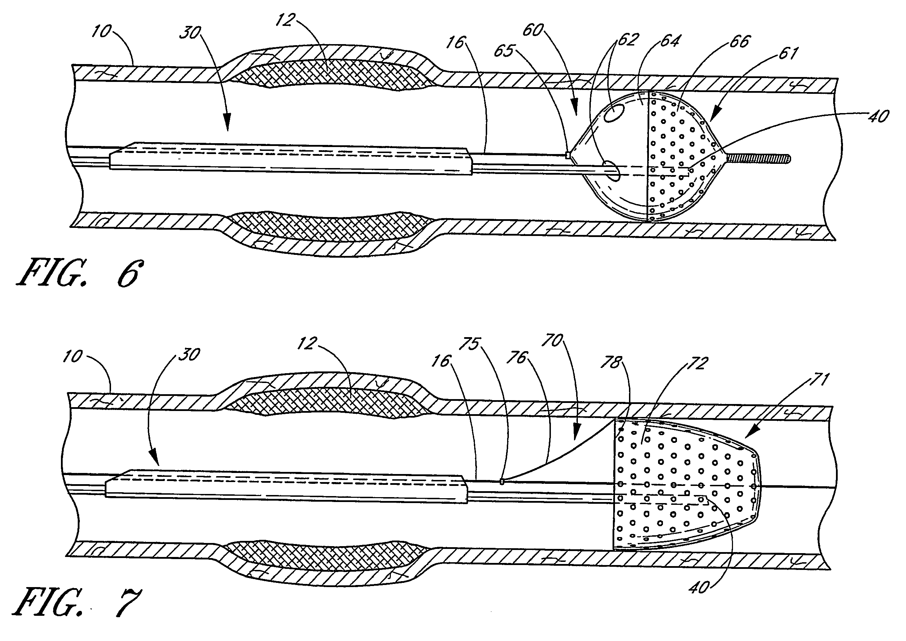

**FIGURE 6** illustrates use of aspiration catheter 30 in combination with alternative filter guidewire 60 comprising guidewire 16 having filter 61 mounted along the distal end portion. Filter 61 has proximal face 64 and distal face 66 that may be mounted on a plurality of struts (not shown). Distal face 66 is preferably formed of a substantially blood-permeable material. Proximal face 64 is formed with a plurality of large inlet holes 62. Aspiration catheter 30 may be used to remove embolic particles 24 that have collected on or around inlet holes 62 or within filter 61. It will be appreciated that the structure of aspiration catheter 30 advantageously allows aspiration port 40 to be advanced beyond filter hub 65. Accordingly, aspiration port 40 may be advanced into the interior volume of filter 61 through one of inlet holes 62. Before, during, or after aspiration port 40 has been advanced into the interior volume, a negative pressure is applied to the proximal end of the aspiration lumen such that particles may be removed proximal to filter 61 or from within the interior volume of filter 61 through aspiration port 40. Optionally, after aspiration port 40 has been inserted through one of inlet holes 62, then aspiration catheter 30 can be retracted, rotated and re-advanced to insert aspiration port 40 into another one of inlet holes 62. Continuous or step-wise aspiration during such manipulation can provide a thorough evacuation of embolic particles from all portions of the interior volume of filter 61.

In various configurations, a vascular filter, such as filter 61 illustrated in **FIGURE 6**, may have proximal inlet holes 62 ranging in size from about 0.5 to 2.0mm diameter or larger. It will be appreciated that the diameter of the distal segment of aspiration catheter 30 should be selected to be smaller in diameter than proximal inlet holes 62 to facilitate entry of aspiration port 40 into the interior volume of the filter. Filter 61 may be self-expanding or may be mechanically deployable, such as by using a pull wire.

**FIGURE 7** illustrates the aspiration catheter in combination with another embodiment of filter guidewire 70 comprising filter 71 having a "basket-shaped" configuration mounted along the distal end of guidewire 16. Filter 71 comprises hoop member 78 at the end of strut 76. Hoop member 78 supports blood-permeable element 72 adapted for capturing and containing embolic particles 24. However, in alternative embodiments, it will be appreciated that element 72 need not be blood-permeable. Strut 76 attaches at the proximal end to guidewire 74 and supports hoop member 78. Strut 76 attaches to guidewire 16 at proximal filter hub 75. Hoop member 78 provides flexibility for adapting to the particular shape of the blood vessel at the treatment site. In one embodiment, filter 70 may be deployed and collapsed using an external sheath (not shown). As illustrated, the structure of aspiration catheter 30 advantageously allows aspiration port 40 to be advanced beyond filter hub 75. Accordingly, aspiration port 40 may be advanced into the interior volume of filter 71 for removal of embolic particles there from.

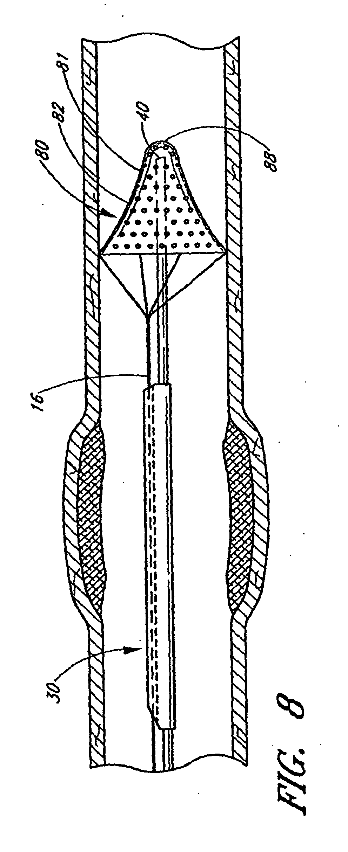

**FIGURE 8** illustrates the aspiration catheter in combination with yet another embodiment of filter guidewire 80 wherein filter 81 is well-suited for use in conjunction with aspiration catheter 30. Filter 81 illustrated in **FIGURE 8** is substantially conical-shaped blood permeable element 82 mounted along the distal end of guidewire 16. The conical shape may be eccentrically located with respect to guidewire 16 such that apex region 88 of blood permeable element 82 is substantially aligned with aspiration port 40 of aspiration catheter 30. The conical shape advantageously urges embolic particles to migrate toward apex region 88 of blood permeable element 82. When particles are located in apex region 88, the particles are more easily drawn into aspiration port 40 and are removed from blood permeable element 82 through aspiration lumen 34. In one embodiment, filter 81 may be self-expanding and delivered using an external sheath.

In addition to the particular embodiment illustrated in **FIGURE 8**, other similarly shaped blood-permeable or substantially occlusive devices may be substituted for use with the aspiration catheter. For example, an occlusive device may be provided wherein the walls are non-permeable and only the apex region is formed with a permeable section to further enhance the migration of particles into the apex region. In another embodiment, the entire occlusive device may be formed of a non-permeable material.

As illustrated in the above embodiments, various embodiments of an aspiration catheter constructed in accordance the present invention may be used in combination with a wide of variety of vascular filters for removal of particles there from. Accordingly, embodiments of the aspiration catheter provide the ability to reestablish the perfusion of blood through the filter such that particles proximal to the filter can be captured within the interior volume. In one method of operation, it may be desirable to wait for the filter to become fully clogged, or substantially fully clogged, before applying a source of negative pressure to the aspiration lumen. This is because aspiration may be more effective once blood flow in the vessel has stopped or has significantly slowed. The catheter body can be advanced relative to the filter until the aspiration port is either located just proximal of the proximal opening in the filter, or into the interior volume of the filter. An aspiration flow is established to remove some or all of the particles from the filter to reestablish perfusion.

In yet another preferred mode of operation, the aspiration catheter may be used with a fully clogged filter to remove particles from the blood vessel in the region proximal to the filter. The catheter body in one embodiment is advanced relative to the filter until the aspiration port is located about 1 to 2 cm proximal of the filter hub. An aspiration flow is established through the aspiration lumen and the catheter body is moved back and forth in a longitudinal direction to aspirate suspended particles from the blood. In this application, it may be advantageous to wait until the filter has become substantially clogged such that the perfusion of blood through the vessel has been reduced or stopped. With little or no perfusion of blood, the suspended particles are relatively stationary and can be easily removed through the aspiration lumen.

Referring now to **FIGURES 9-10A**, aspiration catheter 90 not being part of the invention, comprises elongate body 92 defining single lumen 98 adapted for use as an aspiration lumen and also adapted to slidably receive a guidewire. Elongate catheter body 92 includes side hole 96 along the distal end portion. Single lumen 98 terminates at aspiration port 100 at the distal end. Distal segment 102 of the elongate body is located between side hole 96 and aspiration port 100.

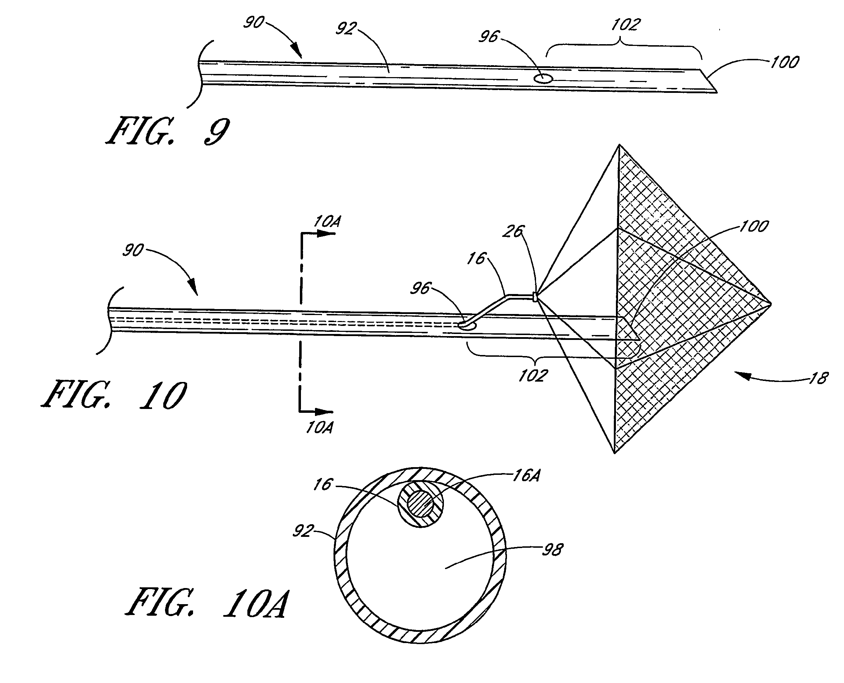

**FIGURE 10** illustrates aspiration catheter 90 being used in combination with a filter guidewire. As shown, the proximal end of guidewire 16 has been received through side hole 96 such that aspiration catheter 90 is advanceable over guidewire 16 through a patient's vasculature. Because distal segment 102 of aspiration catheter 90 does not receive guidewire 16, distal segment 102 is advantageously advanceable beyond proximal hub 26 of filter 18 and into the interior volume of filter 18. **FIGURE 10A** is a cross-sectional view of **FIGURE 10** illustrating catheter body 92 formed with single lumen 98 containing guidewire 16. Lumen 98 is large enough to provide an aspiration flow and also to slidably receive guidewire 16. Illustrated guidewire 16 includes a lumen for receiving pull wire 16A. Pull wire 16A is slidable relative to guidewire 16 for mechanically deploying and collapsing filter 18, as described above.

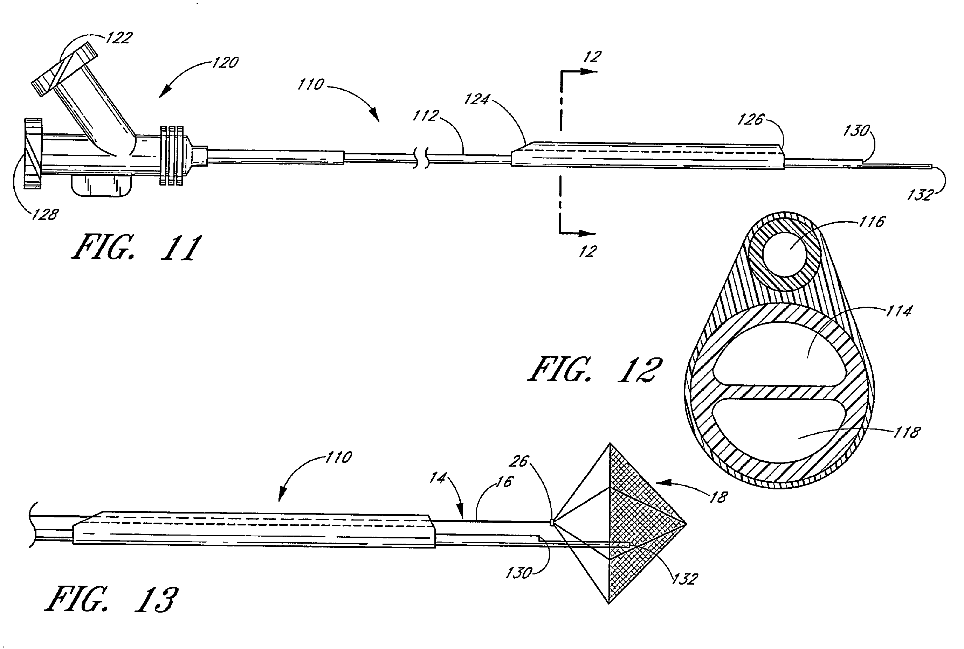

**FIGURES 11** and **12** illustrate another alternative embodiment, aspiration catheter 110, comprising elongate catheter body 112 formed with guidewire lumen 116, irrigation lumen 114 and aspiration lumen 118. Guidewire lumen 116 is adapted for slidably receiving a guidewire and extends between proximal guidewire port 124 and distal guidewire port 126. Irrigation lumen 114 terminates at irrigation port 130. Aspiration lumen 118 terminates at aspiration port 132. In the illustrated embodiment, irrigation port 130 is located distal to distal guidewire port 126 of guidewire lumen 116 and proximal to aspiration port 132. Irrigation fluid may exit through irrigation port 130 to help dislodge and flush out particles located within the interior volume of a filter, thereby facilitating removal of the embolic particles from the filter.

As described above, in the illustrated embodiment, aspiration lumen 118 extends distally beyond irrigation lumen 114. However, it will be appreciated that alternative configurations may also be used. For example, the irrigation and aspiration ports may be located adjacent to one another. In addition, the locations of the irrigation lumen and aspiration lumen may be reversed such that the irrigation lumen extends distally beyond the aspiration lumen. In any case, the irrigation lumen may be used for producing a fluid flow to help dislodge and aspirate particles. In one method of operation, a saline fluid may be forced through irrigation lumen 114 and out through irrigation port 130. In one variation, irrigation port 130 may be shaped to produce a high-speed fluid flow out through the port for enhancing dislodgment of particles. In another method of operation, a clot or plaque dissolving drug or any other medicament well-suited for breaking up, dissolving and/or removing particles from the filter may be delivered through the irrigation lumen to the treatment site.

Referring to **FIGURE 11**, proximal connector 120 is provided at the proximal end of elongate catheter body 112. Proximal connector 120 is provided with first connector 122 for coupling with a source of irrigation fluid (not shown) and second connector 128 for connecting to a source of negative pressure (not shown) for providing an aspiration flow. In one contemplated method of use, the irrigation flow and aspiration flow are each substantially equal in volume for creating a fluid path to effectively dislodge and remove particles from a filter.

**FIGURE 12** illustrates the cross-section of the embodiment shown in FIGURE 11. The cross-sectional view illustrates relative locations of guidewire lumen 116, irrigation lumen 114 and aspiration lumen 118. **FIGURE 13** illustrates the embodiment of **FIGURE 11** in combination vascular filter 18. Aspiration catheter 110 is advanced over guidewire 16 such that aspiration port 132 is located within the interior volume of filter 18. Irrigation port 130 may be located either proximal or distal to filter hub 26.

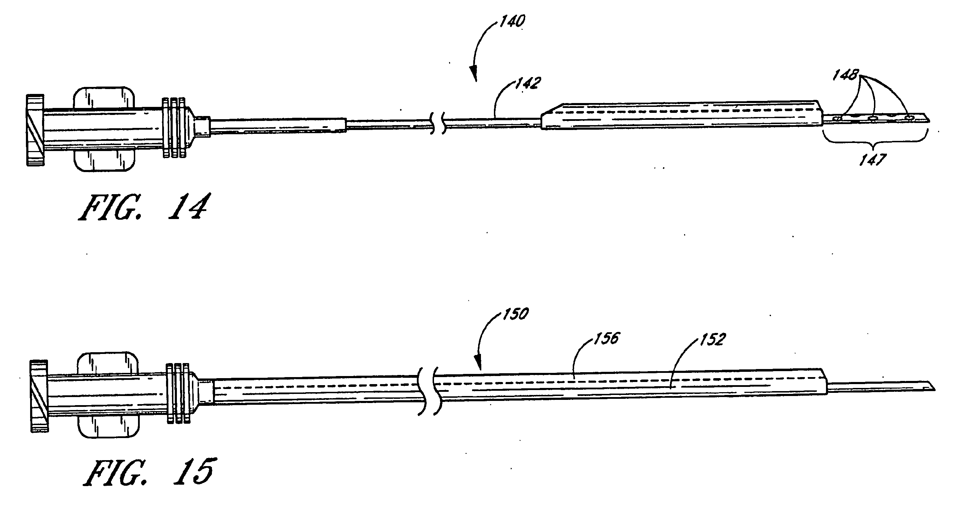

**FIGURE 14** illustrates yet another embodiment, aspiration catheter 140, wherein distal segment 147 of elongate body 142 further comprises a plurality of side ports 148 for providing enhanced aspiration. This embodiment is particularly useful for creating an aspiration flow over a wide area. Variations of this embodiment allow for simultaneous removal of embolic particles from within the interior volume of the filter and also from the region of the blood vessel proximal to the filter.

**FIGURE 15** illustrates another alternative embodiment, aspiration catheter 150, wherein guidewire lumen 156 extends the entire length of elongate catheter body 152 for improved pushability and reduced buckling. The guidewire used in conjunction with this "over-the-wire" embodiment is typically around 300 cm in length to enable the advancement of aspiration catheter 150 over the guidewire.

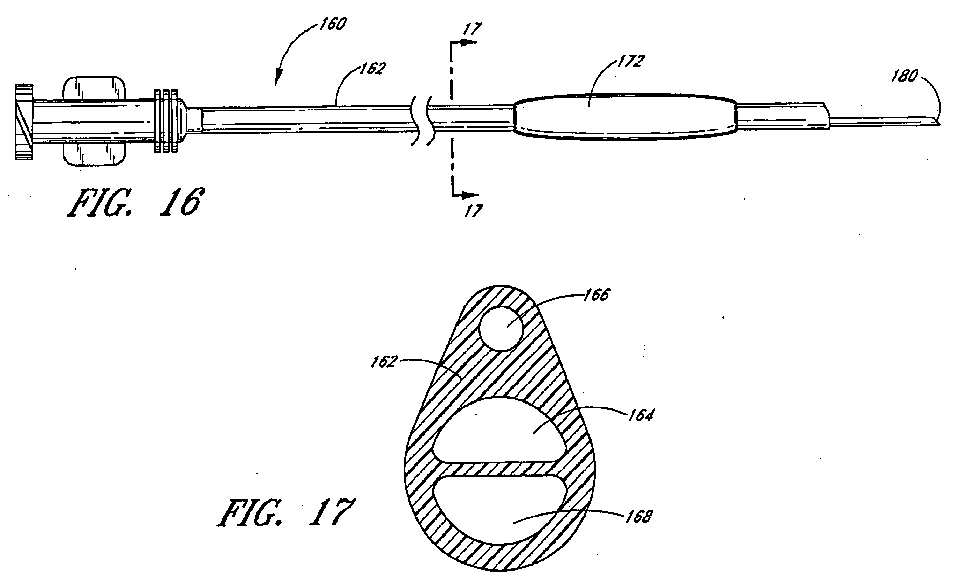

**FIGURES 16 and 17** illustrate yet another alternative embodiment, aspiration catheter 160, further comprising therapy device 172 mounted along a distal end portion of elongate catheter body 162. The illustrated embodiment combines inflatable angioplasty balloon 172 with aspiration lumen 168 into a single integrated device. Although the illustrated embodiment shows a therapy device comprising an angioplasty balloon, it will be understood that other therapy devices may be substituted or added while remaining within the scope of the invention. **FIGURE 17** illustrates the cross-section of elongated catheter body 162. The cross-section illustrates exemplifying locations of aspiration lumen 168, guidewire lumen 166, and inflation lumen 164 along elongate catheter body 162.

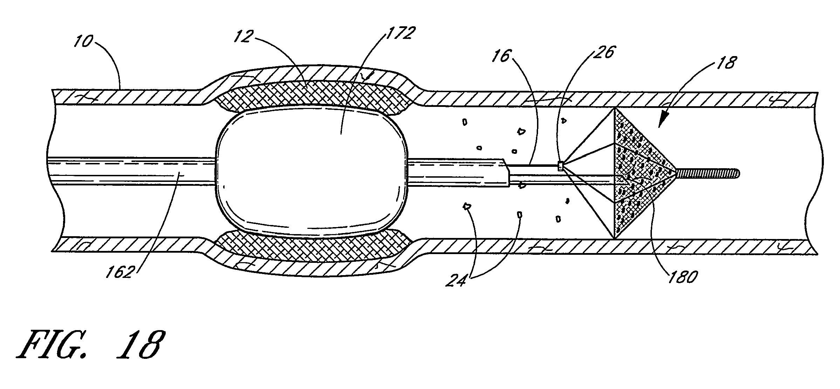

**FIGURE 18** illustrates one method of operation using aspiration catheter 160 of **FIGURE 16** wherein angioplasty balloon 172 has been inflated to dilate occlusion 12 in blood vessel 10. As the vessel wall is stretched and the plaque is partially compressed against the vessel wall during inflation of balloon 172, embolic particles 24 become liberated from the vessel wall and migrate with the flowing blood. As the embolic particles 24 move through blood vessel 10, the particles become trapped and contained within the interior volume of vascular filter 18. While balloon 172 is either inflated or deflated, negative pressure may be applied at the proximal end of the aspiration lumen 168 such that embolic particles 24 are aspirated and removed from filter 18. "Integrated" aspiration catheter 160 advantageously eliminates the need to exchange the therapy device with a separate aspiration catheter to remove particles from the blood vessel and/or filter. In the illustrated embodiment, aspiration catheter 160 and filter 18 have been positioned for performing therapy such that aspiration port 180 is located beyond filter hub 26 at the distal end of guidewire 16 and within the interior volume of filter 18. If aspiration catheter 160 and filter 18 have not been pre-positioned as illustrated in **FIGURE 18**, then balloon 172 should be deflated to allow advancement of aspiration catheter 160 into a position that permits aspiration of embolic particles 24 from within the interior volume of filter 18. The associated method reduces the amount of time that the blood flow is reduced or stopped because the filter will never become fully clogged with material. Furthermore, this method eliminates the trauma to the vessel wall and dislodgment of particles that occurs during exchanging a therapy catheter for an aspiration catheter.

It will be appreciated that certain variations in the aspiration catheter of the present invention and its methods of use and manufacture may suggest themselves to those skilled in the art For example, embodiments of the aspiration catheter may be used for aspirating particles and/or irrigating a treatment site in a wide variety of applications wherein it is desirable to advance a lumen to a location distal to the end of a guidewire. Certain embodiments of the aspiration catheter may be used in conjunction with a wide variety of other medical devices having an interior volume and the operation should not be limited to use with expandable filters. Accordingly, the foregoing detailed description is to be clearly understood as given by way of illustration, the scope of this invention being limited solely by the appended claims.

## Claims

1. An aspiration catheter (30, 110, 140, 150, 160), comprising:
an elongate catheter body (32, 112, 142, 152, 162) having proximal and distal ends;
an aspiration lumen (34, 118, 168) extending longitudinally through the elongate catheter body (32, 112, 142, 152, 162) between the catheter body proximal end and an aspiration port (40, 132, 180) at the catheter body distal end, the aspiration port (40, 132, 180) being sized for aspirating particles from a blood vessel;
a guidewire lumen (36, 116, 156, 166) extending longitudinally through at least a distal end portion of the elongate catheter body (32, 112, 142, 152, 162) adjacent the aspiration lumen (34, 118, 168), the guidewire lumen (36, 116, 156, 166) extending from a proximal port (44, 124) to a distal port (46, 126) and being adapted for slidably receiving a guidewire;
the elongate catheter body (32, 112, 142, 152, 162) includes a distal segment (42, 147) wherein the aspiration lumen (34, 118, 168) extends distally beyond the distal port (46, 126) of the guidewire lumen (36, 116, 156, 166)
**characterized in that**
the distal port (46, 126) of the guidewire lumen (36, 116, 156, 166) opens to the exterior of the elongate catheter body (32, 112, 142, 152, 162).

2. The aspiration catheter (30, 110, 140, 150, 160) of claim 1, wherein the distal segment (42, 147) of the elongate catheter body (32, 112, 142, 152, 162) extends beyond the distal end (46, 126) of the guidewire lumen (36, 116, 156, 166) by about 2 mm to about 30 mm.

3. The aspiration catheter (30, 110, 150, 160) of any one of the preceding claims wherein the aspiration port (40, 180) is formed with an angled tip.

4. The aspiration catheter (140) of any one of the preceding claims, further comprising a plurality of side ports (148) extending through a side wall of the elongate catheter body (142) along the distal segment (147), the side ports (148) adapted for aspirating particles from a blood vessel.

5. The aspiration catheter (30, 110, 140, 160) of any one of the preceding claims, wherein the guidewire lumen (36, 116, 166) is located only along the distal end portion of the elongate catheter body (32, 112, 142, 162).

6. The aspiration catheter (30, 110, 140, 160) of claim 5, wherein the guidewire lumen (36, 116, 166) is about 30 cm or less in length.

7. The aspiration catheter (30, 110, 140, 160) of claim 5, wherein the guidewire lumen (36, 116, 166) is about 6 cm or less in length.

8. The aspiration catheter (110) of any one of the preceding claims, wherein the elongate catheter body (112) further comprises an irrigation lumen (114).

9. The aspiration catheter (160) of any one of the preceding claims, further comprising a therapy device (172) disposed along a distal end portion of the elongate catheter body (162) .

10. An aspiration catheter system comprising:
the aspiration catheter (30, 110, 140, 150, 160) of any one of the preceding claims; a guidewire (16); and a vascular filter (18, 61, 71, 81) disposed along a distal end portion of the guidewire (16).

11. The aspiration catheter system of claim 10, wherein the vascular filter (18, 61,) is self-expanding.

12. The aspiration catheter system of claim 10, wherein the vascular filter (61, 71, 81) is mechanically deployable.

13. The aspiration catheter system of Claim 12, further comprising a pull wire (16A) having a distal end attached to the vascular filter (18, 61) and extending through the guidewire (16), the pull wire (16A) being slidable relative to the guidewire (16) for mechanically deploying the vascular filter (18, 61).

## Patentansprüche

1. Aspirationskatheter (30, 110, 140, 150, 160), der Folgendes umfasst:
einen länglichen Katheterkörper (32, 112, 142, 152, 162), der ein proximales und ein distales Ende hat,
ein Aspirationslumen (34, 118, 168), das sich zwischen dem proximalen Katheterkörperende und einer Aspirationsöffnung (40, 132, 180) am distalen Katheterkörperende in Längsrichtung durch den länglichen Katheterkörper (32, 112, 142, 152, 162) erstreckt, wobei die Aspirationsöffnung (40, 132, 180) zum Aspirieren von Teilchen aus einem Blutgefäß bemessen ist,
ein Führungsdrahtlumen (36, 116, 156, 166), das sich angrenzend an das Aspirationslumen (34, 118, 168) in Längsrichtung durch wenigstens einen distalen Endabschnitt des länglichen Katheterkörpers (32, 112, 142, 152, 162) erstreckt, wobei sich das Führungsdrahtlumen (36, 116, 156, 166) von einer proximalen Öffnung (44, 124) bis zu einer distalen Öffnung (46, 126) erstreckt und dafür eingerichtet ist, verschiebbar einen Führungsdraht aufzunehmen,
wobei der längliche Katheterkörper (32, 112, 142, 152, 162) ein distales Segment (42, 147) einschließt, wobei sich das Aspirationslumen (34, 118, 168) in distaler Richtung über die distale Öffnung (46, 126) des Führungsdrahtlumens (36, 116, 156, 166) hinaus erstreckt,
**dadurch gekennzeichnet, dass**
sich die distale Öffnung (46, 126) des Führungsdrahtlumens (36, 116, 156, 166) zum Äußeren des länglichen Katheterkörpers (32, 112, 142, 152, 162) öffnet.

2. Aspirationskatheter (30, 110, 140, 150, 160) nach Anspruch 1, wobei sich das distale Segment (42, 147) des länglichen Katheterkörpers (32, 112, 142, 152, 162) um etwa 2 mm bis etwa 30 mm über das distale Ende (46, 126) des Führungsdrahtlumens (36, 116, 156, 166) hinaus erstreckt.

3. Aspirationskatheter (30, 110, 150, 160) nach einem der vorhergehenden Ansprüche, wobei die Aspirationsöffnung (40, 180) mit einer abgewinkelten Spitze geformt ist.

4. Aspirationskatheter (140) nach einem der vorhergehenden Ansprüche, der ferner mehrere Seitenöffnungen (148) umfasst, die sich längs des distalen Segments (147) durch eine Seitenwand des länglichen Katheterkörpers (142) erstrecken, wobei die Seitenöffnungen (148) dafür eingerichtet sind, Teilchen aus einem Blutgefäß abzusaugen.

5. Aspirationskatheter (30, 110, 140, 160) nach einem der vorhergehenden Ansprüche, wobei das Führungsdrahtlumen (36, 116, 166) nur längs des distalen Endabschnitts des länglichen Katheterkörpers (32, 112, 142, 162) angeordnet ist.

6. Aspirationskatheter (30, 110, 140, 160) nach Anspruch 5, wobei das Führungsdrahtlumen (36, 116, 166) in der Länge etwa 30 cm oder weniger beträgt.

7. Aspirationskatheter (30, 110, 140, 150, 160) nach Anspruch 5, wobei das Führungsdrahtlumen (36, 116, 166) in der Länge etwa 6 cm oder weniger beträgt.

8. Aspirationskatheter (110) nach einem der vorhergehenden Ansprüche, wobei der längliche Katheterkörper (112) ferner ein Bewässerungslumen (114) umfasst.

9. Aspirationskatheter (160) nach einem der vorhergehenden Ansprüche, der ferner eine Therapievorrichtung (172) umfasst, die längs eines distalen Endabschnitts des länglichen Katheterkörpers (162) angeordnet ist.

10. Aspirationskathetersystem, das Folgendes umfasst:
den Aspirationskatheter (30, 110, 140, 150, 160) nach einem der vorhergehenden Ansprüche, einen Führungsdraht (16) und einen Gefäßfilter (18, 61, 71, 81), der längs eines distalen Endabschnitts des Führungsdrahtes (16) angeordnet ist.

11. Aspirationskathetersystem nach Anspruch 10, wobei der Gefäßfilter (18, 61) selbstexpandierend ist.

12. Aspirationskathetersystem nach Anspruch 10, wobei der Gefäßfilter (61, 71, 81) mechanisch entfaltet werden kann.

13. Aspirationskathetersystem nach Anspruch 12, das ferner einen Zugdraht (16A) umfasst, der ein am Gefäßfilter (18, 61) befestigtes distales Ende hat und sich durch den Führungsdraht (16) erstreckt, wobei der Zugdraht (16A) im Verhältnis zum Führungsdraht (16) verschoben werden kann, um den Gefäßfilter (18, 61) mechanisch zu entfalten.

## Revendications

1. Cathéter d'aspiration (30, 110, 140, 150, 160), comprenant :
un corps de cathéter allongé (32, 112, 142, 152, 162) ayant une extrémité proximale et une extrémité distale ;
un lumen d'aspiration (34, 118, 168) s'étendant longitudinalement à travers le corps de cathéter allongé (32, 112, 142, 152, 162) entre l'extrémité proximale du corps de cathéter et un orifice d'aspiration (40, 132, 180) à l'extrémité distale du corps de cathéter, l'orifice d'aspiration (40, 132, 180) ayant une taille propre pour l'aspiration de particules depuis un vaisseau sanguin ;
un lumen de fil-guide (36, 116, 156, 166) s'étendant longitudinalement à travers au moins une portion d'extrémité distale du corps de cathéter allongé (32, 112, 142, 152, 162) adjacente au lumen d'aspiration (34, 118, 168), le lumen de fil-guide (36, 116, 156, 166) s'étendant depuis un orifice proximal (44, 124) jusqu'à un orifice distal (46, 126) et étant adapté pour recevoir en coulissement un fil-guide ;
le corps de cathéter allongé (32, 112, 142, 152, 162) inclut un segment distal (42, 147) dans lequel le lumen d'aspiration (34, 118, 168) s'étend en direction distale au-delà de l'orifice distal (46, 126) du lumen de fil-guide (36, 116, 156, 166),
**caractérisé en ce que**
l'orifice distal (46, 126) du lumen de fil-guide (36, 116, 156, 166) s'ouvre à l'extérieur du corps de cathéter allongé (32,112,142,152, 162).

2. Cathéter d'aspiration (30, 110, 140, 150, 160) selon la revendication 1, dans lequel le segment distal (42, 147) du corps de cathéter allongé (32, 112, 142, 152, 162) s'étend au-delà de l'extrémité distale (46, 126) du lumen de fil-guide (36, 116, 156, 166) sur environ 2 mm à environ 30 mm.

3. Cathéter d'aspiration (30, 110, 150, 160) selon l'une quelconque des revendications précédentes, dans lequel l'orifice d'aspiration (40, 180) est formé avec un embout en angle.

4. Cathéter d'aspiration (140) selon l'une quelconque des revendications précédentes, comprenant en outre une pluralité d'orifices latéraux (148) s'étendant à travers une paroi latérale du corps de cathéter allongé (142) le long du segment distal (147), les orifices latéraux (148) étant adaptés pour aspirer des particules depuis un vaisseau sanguin.

5. Cathéter d'aspiration (30, 110, 140, 160) selon l'une des revendications précédentes, dans lequel le lumen de fil-guide (36, 116, 166) est situé uniquement le long de la portion d'extrémité distale du corps de cathéter allongé (32, 112, 142,162).

6. Cathéter d'aspiration (30, 110, 140, 160) selon la revendication 5, dans lequel le lumen de fil-guide (36, 116, 166) a une longueur d'environ 30 cm ou moins.

7. Cathéter d'aspiration (30, 110, 140, 160) selon la revendication 5, dans lequel le lumen de fil-guide (36, 116, 166) a une longueur d'environ 6 cm ou moins.

8. Cathéter d'aspiration (110) selon l'une quelconque des revendications précédentes, dans lequel le corps de cathéter allongé (112) comprend en outre un lumen d'irrigation (114).

9. Cathéter d'aspiration (160) selon l'une quelconque des revendications précédentes, comprenant en outre un dispositif de thérapie (172) disposé le long d'une portion d'extrémité distale du corps de cathéter allongé (162).

10. Système à cathéter d'aspiration, comprenant :
le cathéter d'aspiration (30, 110, 140, 150, 160) selon l'une quelconque des revendications précédentes ; un fil-guide (16) ; et un filtre vasculaire (18, 61, 71, 81) disposé le long d'une portion d'extrémité distale du fil-guide (16).

11. Système à cathéter d'aspiration selon la revendication 10, dans lequel le filtre vasculaire (18, 61) est à auto-expansion.

12. Système à cathéter d'aspiration selon la revendication 10, dans lequel le filtre vasculaire (61, 71, 81) est déployable par voie mécanique.

13. Système à cathéter d'aspiration selon la revendication 12, comprenant en outre un fil de traction (16A) ayant une extrémité distale attachée au filtre vasculaire (18, 61) et s'étendant à travers le fil-guide (16), le fil de traction (16A) étant capable de coulisser par rapport au fil-guide (16) pour déployer le filtre vasculaire (18, 61) par voie mécanique.
